# EUROPEAN PATENT APPLICATION

(11) **EP 4 442 225 A2**
(43) Date of publication of application: **09.10.2024**
(21) Application number: 24173540.6
(22) Date of filing: 02.01.2019
(51) Int. Cl.: A61F 2/24

(54) **STENT FEATURES TO AID WITH APPOSITION AND ALIGNMENT TO NATIVE ANATOMY MITIGATION OF PARAVALVULAR LEAK**

(30) Priority: 02.01.2018 US 201862612836 P; 31.12.2018 US 201816237004
(62) Divisional of application: 19736123.1
(71) Applicant: 4C Medical Technologies, Inc., Maple Grove MN 55311 (US)
(72) Inventor: KRUSE, Steven D., Brooklyn Park, 55428 (US); CHAMBERS, Jeffrey W., Brooklyn Park, 55428 (US); DIEDERING, Jason S., Brooklyn Park, 55428 (US); KUMAR, Saravana B., Brooklyn Park, 55428 (US)
(74) Representative: Westphal, Mussgnug & Partner, Patentanwälte mbB

(57) **Abstract**

A collapsible and expandable stent is described that is adapted to treat blood flow regurgitation in a mitral valve or a tricuspid valve of a heart by allowing downstream blood flow and preventing upstream blood flow. The stent comprises an outer section comprising a first stent cell pattern, and an inner valve support section extending radially upward into the outer section and comprising a second stent cell pattern. The inner valve support section supports prosthetic valve leaflets attached therein. The stent further comprises a transition section between the outer section and the inner valve support section comprising a third stent cell pattern that is different from the first stent cell pattern and the second stent cell pattern. The transition section turns radially inward to give rise to a radially upward extension of the inner valve support section within the outer section. The transition section further comprises a plurality of curvilinear struts comprising a degree of curvature and/or twist. A spacing between adjacent curvilinear struts defines the third stent cell pattern of the transition section. Adjacent curvilinear struts are configured to nest together when the stent is collapsed.

## Description

### FIELD OF THE INVENTION

The invention relates to devices and methods for creating optimal apposition and alignment of a support structure or stent of a prosthetic heart valve to treat cardiac mitral or tricuspid valve regurgitation, mitigating paravalvular leak and optimizing functional efficiency of the prosthetic heart valve.

### DESCRIPTION OF THE RELATED ART

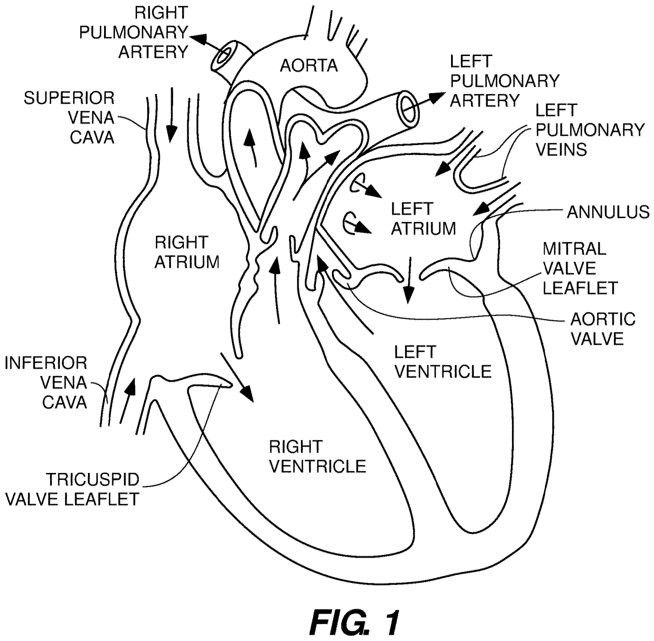

The human heart comprises four chambers and four heart valves that assist in the forward (antegrade) flow of blood through the heart. The chambers include the left atrium, left ventricle, right atrium and left ventricle. The four heart valves include the mitral valve, the tricuspid valve, the aortic valve and the pulmonary valve. Figure 1 illustrates the basic structural features and related blood flow, indicated by arrows, within the human heart.

The mitral valve is located between the left atrium and left ventricle and helps control the flow of blood from the left atrium to the left ventricle by acting as a one-way valve to prevent backflow into the left atrium. Similarly, the tricuspid valve is located between the right atrium and the right ventricle, while the aortic valve and the pulmonary valve are semilunar valves located in arteries flowing blood away from the heart. The valves are all one-way valves, with leaflets that open to allow forward (antegrade) blood flow. The normally functioning valve leaflets close under the pressure exerted by reverse blood to prevent backflow (retrograde) of the blood into the chamber it just flowed out of.

Native heart valves may be, or become, dysfunctional for a variety of reasons and/or conditions including but not limited to disease, trauma, congenital malformations, and aging. These types of conditions may cause the valve structure to either fail to properly open (stenotic failure) and/or fail to close properly (regurgitant).

Mitral valve regurgitation is a specific problem resulting from a dysfunctional mitral valve. Mitral regurgitation results from the mitral valve allowing at least some retrograde blood flow back into the left atrium from the left ventricle. This backflow of blood places a burden on the left ventricle with a volume load that may lead to a series of left ventricular compensatory adaptations and adjustments, including remodeling of the ventricular chamber size and shape, that vary considerably during the prolonged clinical course of mitral regurgitation.

A similar problem may occur when the tricuspid valve weakens or begins to fail. The tricuspid valve separates the right atrium and the right ventricle. Tricuspid regurgitation, also known as tricuspid insufficiency, occurs when the tricuspid valve doesn't close properly, causing blood to flow back up into the right atrium when the right ventricle contracts. Various embodiments of the present invention discussed herein may apply to treatment of mitral valve regurgitation or tricuspid valve regurgitation. Further, embodiments of the present invention may be used to treat and/or prevent mitral and/or tricusid stenosis caused by calcification. The present invention may be used to crack and/or open the stenosis to improve and/or maintain blood flow through the valve.

Native heart valves generally, i.e., mitral valves or tricuspid valves, therefore, may undergo functional repair, including a partial or complete replacement using known methods and devices. Such intervention may take several forms including open heart surgery or open heart implantation of a replacement heart valve. See e.g., U.S. Pat. No. 4,106,129 (Carpentier), for a procedure that is highly invasive, fraught with patient risks, and requiring not only an extended hospitalization but also a highly painful recovery period.

Less invasive methods and devices for replacing a dysfunctional heart valve are also known and involve percutaneous access and catheter-facilitated delivery of the replacement valve. Most of these solutions involve a replacement heart valve attached to a structural support such as a stent, commonly known in the art, or other form of wire network designed to expand upon release from a delivery catheter. See, e.g., U.S. Pat. No. 3,657,744 (Ersek); U.S. Pat. No. 5,411,552 (Andersen). The self-expansion variants of the supporting stent assist in positioning the valve, and holding the expanded device in position, within the subject heart chamber or vessel. This self-expanded form also presents problems when, as is often the case, the device is not properly positioned in the first positioning attempt and, therefore, must be recaptured and positionally adjusted. This recapturing process in the case of a fully, or even partially, expanded device requires re-collapsing the device to a point that allows the operator to retract the collapsed device back into a delivery sheath or catheter, adjust the inbound position for the device and then re-expand to the proper position by redeploying the positionally adjusted device distally out of the delivery sheath or catheter. Collapsing the already expanded device is difficult because the expanded stent or wire network is generally designed to achieve the expanded state which also resists contractive or collapsing forces.

Besides the open heart surgical approach discussed above, gaining access to the valve of interest is achieved percutaneously via one of at least the following known access and delivery routes: femoral access, venous access, trans-apical, trans-aortic, trans-jugular, trans-caroticd, trans-septal, trans-atrial, retrograde from the aorta delivery techniques.

Generally, the art is focused on systems and methods that, using one of the above-described known access routes, allow a partial delivery of the collapsed valve device, wherein one end of the device is released from a delivery sheath or catheter and expanded for an initial positioning followed by full release and expansion when proper positioning is achieved. See, e.g., U.S. Pat. Nos. 8,852,271 (Murray, III); 8,747,459 (Nguyen); 8,814,931 (Wang); 9,402,720 (Richter); 8,986,372 (Murray, III); and 9,277,991 (Salahieh); and U.S. Pat. Pub. Nos. 2015/0272731 (Racchini); and 2016/0235531 (Ciobanu).

However, known delivery systems, devices and methods still suffer from significant flaws in delivery methodology including, *inter alia,* positioning and recapture capability and efficiency.

In addition, known "replacement" prosthetic heart valves are intended for full replacement of the native heart valve. Therefore, these replacement heart valves physically engage tissue within the annular throat, i.e., below the annular plane and upper annular surface, and/or valve leaflets, thereby eliminating all remaining functionality of the native valve and making the patient completely reliant on the replacement valve. Generally speaking, it is a preferred solution that maintains and/or retains the native function of a heart valve, thus supplementation of the valve is preferred rather than full replacement. Obviously, there will be cases when native valve has either lost virtually complete functionality before the interventional implantation procedure, or the native valve continues to lose functionality after the implantation procedure. The preferred solution is delivery and implantation of a valve device that will function both as an adjunctive and/or supplementary functional valve as well as be fully capable of replacing the native function of a valve that has lost, or will lose, most or all of its functionality. However, the inventive solutions described *infra* will apply generally to all types and forms of heart valve devices, unless otherwise specified.

Further, known solutions for, e.g., the mitral valve replacement systems, devices and methods require 2-chamber solutions, i.e., there is involvement and engagement of the implanted replacement valve device in the left atrium and the left ventricle. Generally, these solutions include a radially expanding stent in the left atrium, with anchoring or tethering (disposed downward through the native annulus or annular throat) connected from the stent device down through the annular throat, with the sub-annular surface within the left ventricle, the left ventricular chordae tendineae and even into the left ventricle wall surface(s). *See, e.g.,* the MitraClip^{®} marketed by the Abbott Group and currently the only US approved repair device. With the MitraClip^{®} a catheter containing the MitraClip^{®} is inserted into the femoral vein. The device enters the heart through the inferior vena cava to the right atrium and delivered trans-septally. The MitraClip^{®} passes through the annulus into the left ventricle and sits below the leaflets, clipping the leaflets to decrease regurgitation.

Such 2-chamber and native annulus solutions are unnecessary bulky and therefore more difficult to deliver and to position/recapture/reposition from a strictly structural perspective. Further, the 2-chamber solutions present difficulties in terms of making the ventricular anchoring and/or tethering connections required to hold position. Moreover, these solutions interfere with the native valve functionality as described above because the device portions that are disposed within the left ventricle must be routed through the native annulus and/or annular throat and native mitral valve, thereby disrupting any remaining coaptation capability of the native leaflets. In addition, the 2-chamber solutions generally require an invasive anchoring of some of the native tissue, resulting in unnecessary trauma and potential complication.

It will be further recognized that the 2-chamber mitral valve solutions require sub-annular and/or ventricular engagement with anchors, tethers and the like precisely because the atrial portion of the device fails to adequately anchor itself to the atrial chamber and/or upper portion of the annulus. Again, some of the embodiments, or portions thereof, described herein are readily applicable to single or 2-chamber solutions, unless otherwise indicated.

Finally, known prosthetic cardiac valves consist of two or three leaflets that are arranged to act as a one-way valve, permitting fluid flow therethrough in the antegrade direction while preventing retrograde flow. The native mitral valve is located retrosternally at the fourth costal cartilage, consisting of an anterior and posterior leaflet, chordae tendineae, papillary muscles, ventricular wall and annulus connected to the atria. Each native leaflet is supported by chordae tendineae that are attached to papillary muscles which become taut with each ventricular contraction preserving valvular competence. Both the anterior and posterior leaflets of the native valve are attached via primary, secondary and tertiary chordae to both the antero-lateral and posterio-medial papillary muscles. A disruption in either papillary muscle in the setting of myocardial injury, can result in dysfunction of either the anterior or posterior leaflet of the mitral valve. Other mechanisms may result in failure of one, or both of the native mitral leaflets. In the case of a single mitral valve leaflet failure, the regurgitation may take the form of a non-central, eccentric jet of blood back into the left atrium. Other leaflet failures may comprise a more centralized regurgitation jet. Known prosthetic valve replacements generally comprise leaflets which are arranged to mimic the native valve structure, which may over time become susceptible to similar regurgitation outcomes.

As discussed above, known delivery methods and devices comprise expandable prosthetic valves that are collapsed during delivery via a delivery catheter. The problems with such collapsing and expanding structures include placing strain on the regions of the structure, e.g., stent, that must bend to accommodate the collapsing and expanding states. Further, the collapsed geometry in known devices may not be controlled or predictable, adding to the strain on the collapsing and expanding structure elements.

In addition, known prosthetic mitral valves may be improved upon in terms of sealing and protecting against paravalvular leakage from the left ventricle to the left atrium as well as the attachment and alignment of the leaflets to the support structure.

Various embodiments of the present invention address these, *inter alia*, issues.

### BRIEF SUMMARY OF THE INVENTION

An expandable and collapsible stent comprising prosthetic leaflets attached to an inner valve support section that extends radially upward into an outer stent section, the stent adapted for use in treating a dysfunctional native heart valve, including the mitral valve, the tricuspid valve and the aortic valve. A transition stent section is disposed between the inner valve support section and the outer stent section. Each of the outer stent section, the inner valve support section and the transition stent section may comprise struts or the equivalent that form and define cells having a pattern, wherein each section may comprise a different cell pattern. Transition stent section preferably comprises struts that are of equal curvature, with adjacent struts equally spaced from each other to allow nested collapsing of the transition stent section struts. A boss section extending downstream away from the inner valve support section may be provided attached to, or integrated with, the outer stent section or the transition stent section to aid in alignment and retention of the expanded stent and may comprise a shape that is complementary to a heart chamber annulus.

### BRIEF DESCRIPTION OF THE DRAWINGS

FIG. 1 is a cross-sectional view of the human heart and related blood vessels and valves.
FIG. 2 is a perspective view of one embodiment of the present invention.
FIG. 3A is a bottom view of one transition section embodiment of the present invention.
FIG. 3B is a bottom view of one transition section embodiment of the present invention.
FIG. 3C is a bottom view of one transition section embodiment of the present invention.
FIG. 4A is a bottom view of one transition section embodiment under compression of the present invention.
FIG. 4B is a bottom view of one transition section embodiment under compression of the present invention.
FIG. 5 is a side cutaway view of a mitral valve annulus.
FIG. 6 is a bottom cutaway view of one embodiment of the present invention.
FIG. 7 is a side view of one embodiment of the present invention.
FIG. 8A is a perspective view of one embodiment of the present invention.
FIG. 8B is a perspective view of one embodiment of the present invention.
FIG. 9 is a side view of one embodiment of the present invention.
FIG. 10 is a bottom view of one embodiment of the present invention.
FIG. 11 is a side cutaway view of one embodiment of the present invention.
FIG. 12 is a side cutaway view of one embodiment of the present invention.

### DETAILED DESCRIPTION OF THE INVENTION

While the invention is amenable to various modifications and alternative forms, specifics thereof are shown by way of example in the drawings and described in detail herein. It should be understood, however, that the intention is not to limit the invention to the particular embodiments described. On the contrary, the intention is to cover all modifications, equivalents, and alternatives falling within the spirit and scope of the invention.

The following description refers generally throughout to the anatomical structures and related blood flow illustrated in Fig. 1.

Generally, various embodiments of the present invention are directed to devices and methods for creating optimal apposition of a support structure or stent of a prosthetic heart valve to treat cardiac mitral or tricuspid valve regurgitation, mitigating paravalvular leak and optimizing functional efficiency of the prosthetic heart valve.

The support structure (e.g., an expandable stent) has multiple functions to aid with the treatment of cardiac valve regurgitation (mitral or tricuspid). These functions include its function as a scaffold for the functioning prosthetic valve and associated leaflets, apposition to the atrial anatomy, optimized radial force for compliance with atrial distension, ability to load and deploy from a minimally invasive delivery system, and geometry to support with mitigating against paravalvular leak (PVL). The design features of the stent are adapted to meet one or more of the functions identified above. Specific design features and attributes for the stents are discussed in detail below.

### Stent Design Concepts

The stent design concepts are intended to support minimally invasive procedures for the treatment of valvular regurgitation - mitral, tricuspid and/or otherwise. The stents may be self-expandable (e.g. nitinol or similar materials) or balloon expandable (e.g. cobalt chromium or similar materials) as is known in the art. The stent may be made of cells that may be open celled diamond like structures or continuous structures that have a working cell element. The stents may also be constructed using tubing, wires, braids or similar structures. Specific design features that aid with the functioning of the stent are described in detail below.

### Stent "Iris" transition cells

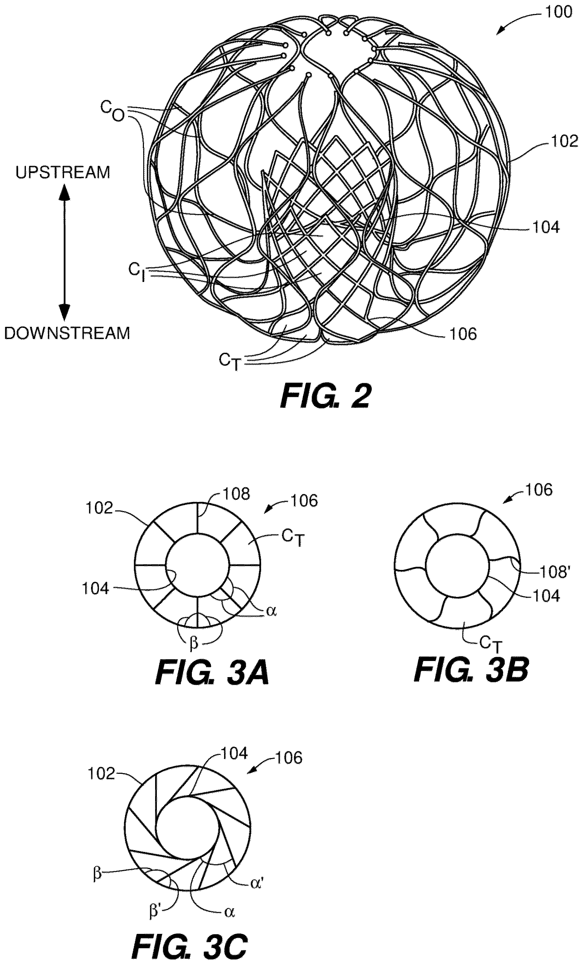

With reference now to Figures 2-3B, one embodiment of the stent 100 of the present invention comprises an outer section 102 - that may generally be circular though need not be a perfectly round circular structure when fully and/or partially expanded - and an inner valve support section 104 - which may be cylindrical but need not be a constant diameter cylinder and is adapted to support and retain prosthetic valve leaflets (not shown in Fig. 2) within the inner valve support section 104, most preferably at a point that located above the native annulus, e.g., the mitral valve annulus, though other attachment points for the prosthetic leaflets are within the scope of the present invention. Further, as discussed above, the stent 100 may be configured to supplement and/or replace the function of the tricuspid valve. A preferred construction comprises the prosthetic leaflets disposed above the native leaflets, wherein the prosthetic leaflets are attached and spaced sufficiently away from (above) the native leaflets so as to not physically interfere or interact with the native leaflets. However, certain embodiments contemplate some interaction with the native leaflets.

Individual cells C_{O} forming the outer section 102 of stent 100 are visible in Figure 2 as open cell regions defined by the material used to form the expandable stent 100.

Individual cells C_{I} forming the inner valve support section 104 are also illustrated as open cells regions formed within an inner region R defined by outer section 102, wherein the inner valve support section extends radially upward into the inner region R. As shown, individual cells C_{I} are of a different size, and may comprise a different shape, than that of individual cells Co.

The region of stent 100 that facilitates the radially inward transition of the stent 100 from the outer section 102 to the inner section 104 of the stent 100 is the transition cell region 106. Transition cell region 106 may comprise cells C_{T} that may comprise a different size and/or shape that either the outer section cells C_{O} and/or the inner section cells C_{I}. The outer and/or inner regions 102, 104, and/or transition cell region 106 of the stent 100 may be constructed from one continuous structure or may combine two or more structures to achieve intended design goals. Transition cell region 106 comprises generally a radially upward turn to allow the inner valve support section 104 to reside within the inner region 102 as shown in Fig. 2. In some embodiments, the lower portion of inner valve support section 104, that is the portion of the inner valve support section 104 that is in connection with the cells C_{T} of transition cell region 106 may also comprise a curving shape to facilitate and/or complete the radially upward turn into the inner region 102.

The geometry and/or shape of the transition cells C_{T} may be substantially straight segments when expanded as in Figure 3A below or may, as shown in Figure 3B, incorporate an offset or a twist in the stent cell pattern when expanded to allow for a controlled compression of the stent. Exemplary cross-sectional geometry of the transition cell region 106 viewed from the bottom of stent 100 is represented schematically in Figures 3A and 3B.

This transition cell region 106 of the stent 100 may be a strut, completed cell section or a partial cell section. The transition cell region 106 may have any number of struts (minimum of 3) or cell sections as generally required to meet design needs. Transition cells C_{T} or struts may be evenly spaced and formed by substantially straight and equally spaced apart struts 108 as shown in Fig. 3A, that extend away from the inner valve support section 104 with equal angles α on both sides of the strut 108 and equal angles β on both sides of strut 108 with respect to its intersection or integration with outer support section 102.

In a preferred embodiment, the struts 108 of transition section 106 may be straight as in Figure 3A, but with non-equal angles relative to the inner valve support section 104 and outer support section 102 as shown in Figure 3C. There, the straight struts 108 are slanted so that a smaller angle α and a larger angle α' are provided relative to the inner valve support section 104. Similarly, a smaller angle β' and a larger angle β are provided relative to the outer support section 102. This allows a compressed nesting of the slanted struts 108 of transition section 106.

In another preferred embodiment, the transition cell region 106 may comprise transition cell struts 108' that comprise transition cells CT that are formed by struts 108' having an offset, i.e., not straight, are twisted and/or curvilinear. The degree of offset and/or twist and/or curvature of the struts 108', and therefore the size and/or shape of the resultant expanded cells CT may be varied dependent on the number of cells/struts in the transition cell region 106, packing density when the stent is collapsed, and stress/strain distribution limitations of the transition cell region 106.

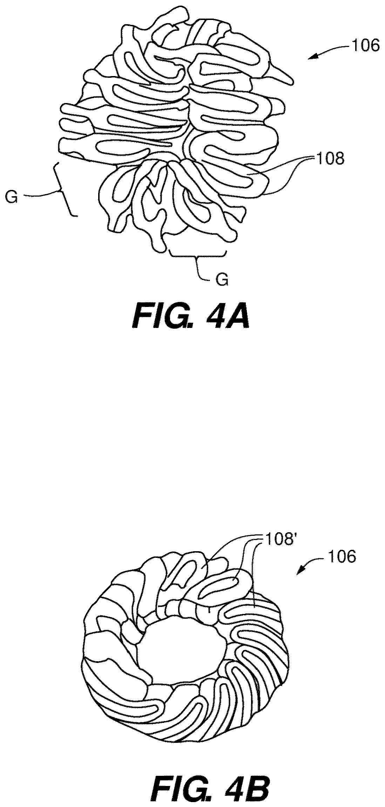

The structure of Figures 3B and 3C are preferred over the straight transition cell region 106 structure of Fig. 3A for several reasons. Figure 4A shows a transition cell region 106 in a collapsed form using the substantially straight struts 108 of Fig. 3A and with, undesirable, gaps G between selected struts 108. Though this resultant gapping collapsed transitional cell region 106 is workable, it is not optimal.

Thus, the transition section 106 of Figure 4B, using e.g., the offset and/or twisted and/or curved plurality of struts 108' of Fig. 3B or the slanted straight struts 108 of Fig. 3C, allows for a controlled and predictable collapsed form of the stent, without gapping between the struts 108'. This, in turn, minimizes the amount of stress/strain concentration at the lower region of the stent 100 during collapsing as is required for delivery of the expandable stent 100 to the heart region of interest. Additionally, the collapse of the cells is also symmetrical and uniform, which could aid with mitigating against damage to the valve tissue or fabric when it is attached to the stent cells. Reduction in overall stress/strain of the transition strut section may benefit the durability of the stent and the valve tissue.

A feature of certain embodiments of the transition cell region 106 as shown in Figures 3B and 3C and 4B, i.e., with offset, twisted and/or curved struts 108' or slanted straight struts 108, is that, as best shown in Figure 3B, the struts 108' each comprise the same offset, twist and/or curvature. This, in turn, enables a close nesting of adjacent struts 108' as the stent 100 is collapsed down for delivery and subsequent expansion.

### Stent geometry - Transition section

The geometry of the native valve annulus is typically D-shaped, saddle shaped or oval depending on the diseased state of the patient. The transition cell region 106 of the stent100 interacts with the valve annulus, e.g., the mitral valve annulus, to ensure that the positioning allows for normal forward flow of blood while preventing regurgitant blood flow from entering the left atrium. The transition cell region 106 of the stent 100 may be located upon expansion either supra-annular, at the annulus or sub-annular while the functioning prosthetic valve stays at or above the native valve annulus, thereby minimizing and, in certain embodiments, eliminating physical interaction between the native valve leaflets and the prosthetic valve leaflets.

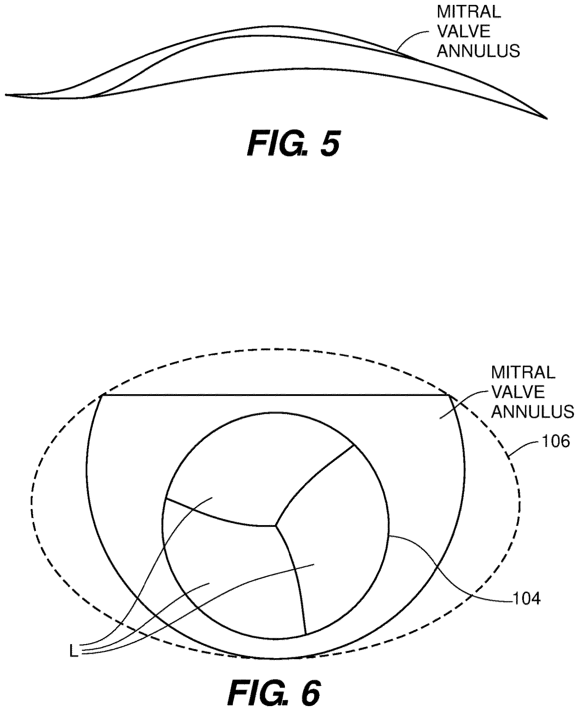

As shown in Fig. 5, below, the exemplary mitral valve annulus may comprise a saddle shape as viewed from the side. Thus, transition cell region 106 may comprise a complementary shape to fit sealingly against the saddle-shaped mitral valve annulus and/or may comprise a degree of compliance that enables the transition cell region 106 to adaptingly seal against the saddle-shaped annulus as the self-expanding stent 100 is allowed to expand within the exemplary left atrium.

Figure 6, viewing the annulus from below the exemplary mitral valve annulus, illustrates the mitral valve annulus having a substantially "D" shaped opening. Thus, the shapes and/or geometry of the transition cell region 106 and the outer stent section 102 are critical in ensuring alignment of the implant to the native valve, and for mitigation of PVL. Figure 6 illustrates the expanded transition cell region 106 in the outer dashed line, extending at least to the boundaries at all locations along the D-shaped mitral valve annulus. In this way, and in some cases in combination with the complementary shaping of transition cell region 106 discussed above in connection with Fig. 5, the transition cell region 106 also assists in the optimization of the positioning and/or alignment of the inner valve support 104, and valve leaflets L attached thereto, over the exemplary mitral valve annulus to help optimize blood flow (and blockage of regurgitant) therethrough. In addition, the transition cell region 106, properly positioned as discussed herein, works to minimize and/or eliminate any paravalvular leakage (PVL) from the left ventricle back into the left atrium.

Any one or more of the design concepts discussed below may be reasonably combined to achieve intended design function.

### Circular/Oval profile

The transition cell region 106 of the stent 100 may be circular or ovalized to provide adequate oversizing of the implant to the native valve annulus. The aspect ratio of the oval may vary to accommodate the dynamics of the native valve annulus. *See*, *e.g.*, Figure 6.

### D-shaped profile

The transition cell region 106 of the stent 100 may be D-shaped to match the shape of or oversized accordingly to the native annulus. The cells of the stent are profiled where either the stent struts or cells may be shape set to retain the D-shape. *See* Fig. 6.

### Boss/extended profile

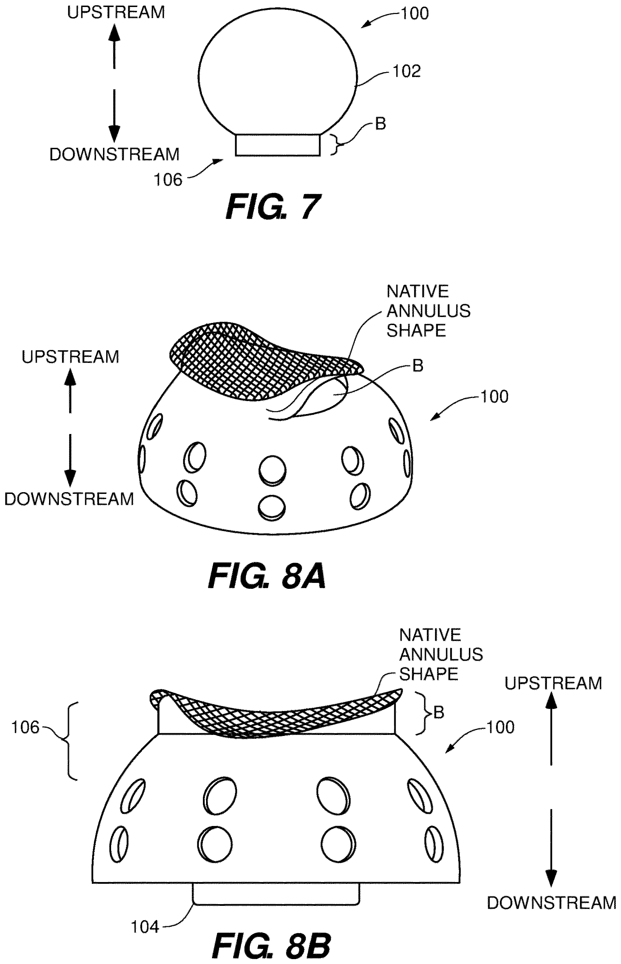

As shown in Figure 7, a boss section B may be provided integrated with, or attached to, the transition cell region 106, wherein the boss section B extends away from the outer stent section 102 in a downstream direction such that the stent seats itself at the level of annulus or subannular, with the boss section B extending downward slightly into the annular throat, and radially expanding in some cases against the inner portion of the annular throat. Alternatively, boss section B may be attached to, or integrated with, outer section 102. The boss section B allows the stent 100 to maintain position/alignment with respect to the native valve leaflets while the transition to the outer stent section 102 may be used to mitigate against PVL. The dimensions of the boss section B (length) may be equal to or less than the maximum diameter (or width) of the outer section 102 of the stent 100 and may be oversized and/or varied to provide radial forces against the portion of the annular throat engaged by boss section B when stent 100 and boss section B are expanded.

The boss section B may, similar to the shaping considerations relating to the transition cell region 106 discussed above in connection with Figures 5 and 6, comprise the following design considerations, whether boss section B is integrated with or attached to transition section 106 or outer section 102 of stent 100:

### Circular/oval profile

The aspect ratio and height of the boss section B may be varied within reasonable limits, and may in some cases extend downward into the annular throat, i.e., sub-annularly positioned. In some embodiments, the boss section B may be slightly oversized and/or varied relative to the native mitral valve orifice or throat, with radial forces pressing on the annular through when the boss section B is expanded to provide additional alignment force and presence as well as PV mitigation.

### D-shaped profile

The boss section B may comprise a D-shape to match the D-shaped exemplary mitral valve annulus, in some embodiments, the length of the D-shaped boss section B may be varied to match, or oversize, the annulus dimensions.

### 3D saddle shape

The profile of the extended boss section B whether attached to or integrated with transition section 106 or outer section 102 may be varied in three dimensions to match with the saddle-shaped profile of the native annulus. The height/depth of the boss section may be contoured to vary along the length and/or width of the profile to form a complementary profile and fit for the native annulus. For example, for the D-shaped boss section B, the ends of the boss section B may have more depth/height as compared to closer to the center to form the desired complementary shaping. This may allow the profile of the stent 100 to sit well into the commissures for alignment as well as mitigation of PVL. In some embodiments, only the ends with greater depth/height may extend into the native annular throat, i.e., subannularly. A representation of such a profile is provided in Figures 8A and 8B which are inverted relative to the other stent figures to provide better view of the boss section B. The mesh-like structure is depicting the native upper annular surface shape, with complementary contouring of the boss section B to provide a relatively fit to assist in aligning the stent 100 as well as prevention of PVL.

### Flared profile

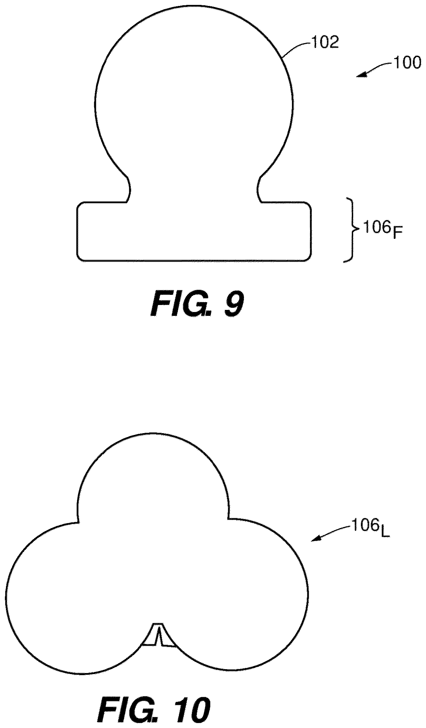

The boss section B, whether attached to or integrated with transition cell section 106 or outer section 102 of the stent 100 may be flared to seat itself at the level of the native exemplary mitral annulus and/or subannular, i.e., at least partially disposed below the upper annular surface and therefore within the annular throat, for positioning and alignment of the stent 100, and in particular the prosthetic leaflets L, with respect to the native valve leaflets. The diameter (or length) of the flared transition cell region 106_{F} may be equal to or greater than the maximum diameter (or width) of the outer section 102 of the stent. A representation of such a profile is provided in Figure 9. The height/depth of the flared section 106_{F} may be varied to accommodate variability in expected native valve geometries.

### Lobular Profile

The boss section B, whether attached to or integrated with transition cell region 106 or outer section 102 of the stent 100 may be shaped like lobes 106_{L}. A representation of such three-lobe structure is provided in Fig. 10, as viewed from the bottom of the transition cell region 106_{L}. The dimensions of the lobe may be varied to match or accommodate various geometries of the native human valve. A boss/extension B and/or a flare 106_{F} may be added to the lobe(s) of the lobular transition cell region 106_{L} and/or outer section 102. Each of the lobes of the lobular transition cell region 106_{L} may have similar dimensions or may have different dimensions. The ends of the lobes are connected to maintain its continuity. The geometry of the individual and/or all lobes of the lobular transition cell region 106_{L} may be varied (e.g. flat sections at the top of lobes, extended arms of the lobes, non-spherical lobes) to accommodate design needs.

In addition to aiding implant positioning/alignment and mitigation of PVL, the lobular design is useful as a mechanism to reduce the closing volume flow needed for the closure of the implant leaflets. Typically, there is a minimum amount of regurgitant flow or volume across the native leaflets needed for closure of leaflets (either native or implanted valves). The lobes of the stent geometry - similar to a coronary sinus function for aortic valve replacement leaflets - directs flow behind the implanted tissue valve leaflets and aids with closing the leaflets at lower closing volume and/or pressure.

### Stent geometry - Outer section 102

The outer section 102 of the stent 100 assists with engaging the exemplary left atrium by oversizing and prevents embolization and/or migration of the implant. The compliance of the stent 100 and outer section 102 may be tailored to meet with the compliance of the atrial anatomy, and varied to accommodate expected variations in anatomy. The geometries of the outer stent section 102 may be designed to accommodate variabilities of the atrial anatomy.

Specific designs of the outer section 102 are discussed below.

The outer section 102 of the stent 100 may comprise a circular shape that may have a round shape or may comprise a non-round circular shape, whose diameter may be varied to accommodate expected variations in human atrial or other heart chamber anatomy.

The outer section 102 of the stent 100 may be oval with a combination of aspect ratios for the major and minor diameter to accommodate expected variations in human atrial or other heart chamber anatomy.

The bottom section of the stent 100, i.e., the transition cell region 106 and its various configurations discussed above, and/or the boss section B, may be flat, convex, concave or slanted to accommodate expected variations in human atrial or other heart chamber anatomy.

The top of the outer region 102 of the stent 100 may be flat, convex, concave or slanted to promote better contact and apposition to the atrial or other heart chamber anatomy.

### Stent features for attachment

Various features are incorporated into the stent 100 to assist with (but not limited to) valve attachment, load distribution, fabric attachment, repositioning, reorientation, and recapture of the stent 100. Specific features are discussed below.

### Leaflet Extension Attachment Feature (LEAF)

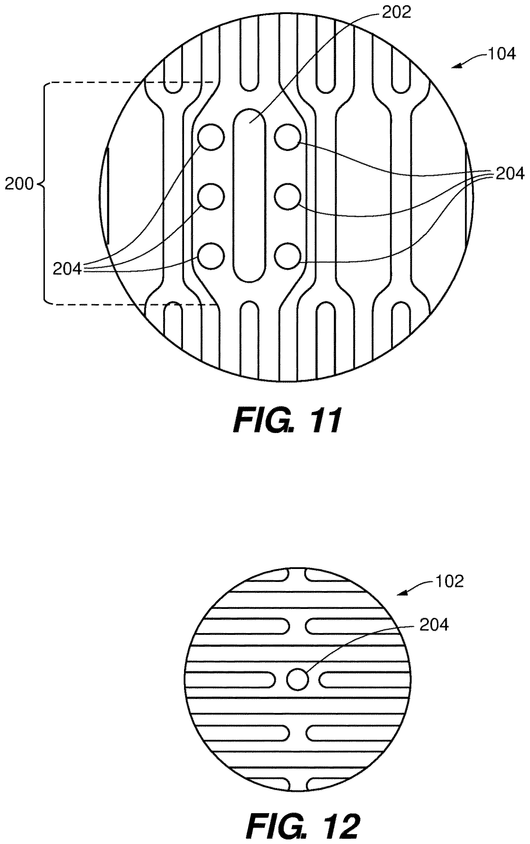

With reference now to Figures 11 and 12, the ends of the leaflets L need to be reliably attached to the stent frame to ensure that the load distribution may occur efficiently between the valve leaflet and the stent. The valve tissue leaflet ends need to be folded over the inner valve support section 104 for attachment. The LEAF 200 is designed to capture these leaflet ends and fold them over the outside of the inner valve support section 104 of the stent 100. The slot 202 at the center of the LEAF is designed to allow the leaflet ends to pass through slot 202 and fold on the outside of the inner valve support section 104 of the stent 100. The holes or apertures or eyelets 204 in the LEAF 200 allow for sutures to attach the valve leaflet L to the stent 100.

### Stent Eyelets/slots

As discussed, one or more eyelets 204 and/or slots 202 may be incorporated into the stent to assist with valve attachment, fabric attachment for PVL mitigation, delivery system attachment for repositioning/reorientation of the implant, and recapture of the implant during or after deployment.

Figure 12 shows a representation of an eyelet feature 204 added to the laser cut profile of the outer section 102 of the stent to aid with fabric attachment. The number and location of the eyelet(s) 204 may vary to accommodate design needs. The size, dimensions, and geometry of the eyelet(s) 204 may also be varied reasonably. These eyelets 204 may also be extended to become slots 202 as needed. These features may be included in any section of the stent 100 - outer section 102, inner valve support section 104 and/or at the transition section 106 - without limitations. The location of the eyelets 204 is generally positioned in between the stent cells C at the junction of struts 108, 108' (also defined as nodes). However, they may be incorporated along the length of the stent struts 108, 108' or added as features in between stent cells as needed.

Representative Embodiments of the present invention comprise:
1. A collapsible and expandable stent comprising:
   an outer section comprising a first stent cell pattern;
   an inner valve support section extending radially upward into the outer section and
   comprising the first stent cell pattern or a second stent cell pattern;
      a transition section between the outer section and the inner valve support section comprising a third stent cell and/or strut pattern that is different from the first stent cell pattern and the second stent cell pattern.
2. The stent of embodiment 1, wherein the third stent cell and/or strut pattern of the transition section further comprises a plurality of curvilinear struts comprising a degree of curvature and/or twist, and a spacing between adjacent curvilinear struts.
3. The stent of embodiment 2, further comprising the degree of curvature and/or twist adapted to be the same for each of the plurality of curvilinear struts.
4. The stent of embodiment 2, further comprising the spacing between adjacent curvilinear struts being constant or equal across the plurality of curvilinear struts.
5. The stent of embodiment 2, further comprising the degree of curvature and/or twist adapted to differ or vary for at least one of the plurality of curvilinear struts.
6. The stent of embodiment 2, further comprising the spacing between adjacent curvilinear struts varying for at least one pair of adjacent curvilinear struts.
7. A method for controlled and predictable compression of a stent according to embodiment 2, comprising:
   radially compressing the outer section and transition section of the stent;
   enabling the plurality of curvilinear struts of the transition section to nest together in a regular and predictable pattern; and
   thereby reducing stress and strain on the transition section.
8. A prosthetic mitral valve comprising the collapsible and expandable stent according to embodiment 1, further comprising the transition section comprising at least one expanded profile selected from the group consisting of: substantially circular, substantially oval or elliptical, or substantially D-shaped.
9. prosthetic mitral valve comprising the collapsible and expandable stent according to embodiment 1, further comprising a boss section integrated with, or attached to, the transition section, wherein the boss section is positioned downstream from the inner valve support, the boss section comprising at least one shape or profile selected from the group consisting of: substantially circular, substantially D-shaped, substantially oval or elliptical, and substantially complementary and/or adaptable to the upper annular shape.
10. The prosthetic mitral valve of embodiment 9, further comprising the boss section extending downstream to a position that is selected from the group consisting of: above the annular plane, substantially co-planar with the annular plane, and slightly below the annular plane within the annulus.
11. The collapsible and expandable stent of embodiment 1, wherein the transition section comprises a flared profile comprising a diameter that is equal to or greater than an expanded maximum diameter of the outer section of the stent and may interact with native tissue selected from the group consisting of: above the annular plane, substantially co-planar with the annular plane, and slightly below the annular plane within the annulus.
12. The collapsible and expandable stent of embodiment 1, wherein the transition section comprises two or three lobe-shaped elements, wherein the number of lobe-shaped elements is matched to the number of native valve leaflets;
   or wherein the lobe-shaped elements maybe continuous with the inner stent.
13. The collapsible and expandable stent of embodiment 12, further comprising the lobe-shaped elements of the transition section adapted to direct regurgitant blood flow across the native valve leaflets in a manner that reduces the closing volume and/or pressure required to close prosthetic leaflets attached within the inner valve support section.
14. The collapsible and expandable stent of embodiment 1, further comprising the outer section having a top section of an expanded shape selected from the group consisting of: flat, convex, concave or slanted.
15. The collapsible and expandable stent of embodiment 1, further comprising: at least two slots and formed by adjacent struts forming the second cell structure pattern of the inner valve support section, each slot adapted for receiving an inner end of a prosthetic valve leaflet therethrough and further adapted to align, and maintain alignment of the prosthetic valve leaflet within the inner valve support section.
16. The collapsible and expandable stent of embodiment 15, further comprising: one or more holes disposed in a strut adjacent the slot, each hole adapted for suturing the inner end of the prosthetic valve thereto.
17. The collapsible and expandable stent of embodiment 1, further comprising:at least two slots defined by the second cell structure pattern of the inner valve support section, each slot formed through a strut of the second cell structure pattern of the inner valve support section and adapted for receiving an inner end of a prosthetic valve leaflet therethrough and further adapted to align, and maintain alignment of the prosthetic valve leaflet within the inner valve support section.
18. The collapsible and expandable stent of embodiment(s) 15 and/or 17, further comprising: one or more holes disposed in a strut adjacent the strut comprising the slot, each hole adapted for suturing the inner end of the prosthetic valve thereto.
19. A collapsible and expandable stent comprising:
   an outer section comprising a cell structure pattern formed at least partially by a plurality of struts; and
   at least one fabric attachment eyelet or aperture defined through at least one of the plurality of struts.
20. A collapsible and expandable stent comprising:
   an outer section comprising a cell structure pattern formed at least partially by a plurality of struts; and
   at least one fabric attachment eyelet or aperture defined between adjacent struts of the plurality of struts.
21. The collapsible and expandable stent according to one or more of embodiments 1, 2, 7, 19 and 20, wherein the stent comprises a prosthetic mitral valve or a prosthetic tricuspid valve.
22. A method for controlled and predictable compression of a stent according to any of the foregoing embodiments, comprising:
   radially compressing the outer section and transition section of the stent;
   enabling the plurality of curvilinear struts of the transition section to nest together in a regular and predictable pattern; and
   thereby reducing stress, strain and/or tears on fabric on stent and/or of the leaflets.

The description of the invention and its applications as set forth herein is illustrative and is not intended to limit the scope of the invention. Features of various embodiments may be combined with other embodiments within the contemplation of this invention. Variations and modifications of the embodiments disclosed herein are possible, and practical alternatives to and equivalents of the various elements of the embodiments would be understood to those of ordinary skill in the art upon study of this patent document. These and other variations and modifications of the embodiments disclosed herein may be made without departing from the scope and spirit of the invention.

Although the present disclosure is not so limited, the following numbered examples demonstrate one or more aspects of the disclosure. Example 1. A collapsible and expandable stent adapted to treat blood flow regurgitation in a native heart valve by allowing downstream blood flow and preventing upstream blood flow, the stent comprising an outer section comprising a first stent cell pattern; an inner valve support section extending radially upward into the outer section and comprising the first stent cell pattern or a second stent cell pattern, the inner valve support section supporting prosthetic valve leaflets attached therein; a transition section between the outer section and the inner valve support section comprising a third stent cell pattern that is different from the first stent cell pattern and the second stent cell pattern, wherein the patterns of the first stent cell pattern, the second stent cell pattern and the third stent cell pattern comprise cell size and cell shape.

Example 2. The stent of example 1, wherein the third stent cell pattern comprises cells having a shape that is different than the cells of the first stent cell pattern and the cells of the second stent cell pattern.

Example 3. The stent of example 1, wherein the inner valve support section comprises the second stent cell pattern and wherein the second stent cell pattern is different from the first stent cell pattern.

Example 4. The stent of example 1, wherein the third stent cell pattern of the transition section further comprises a plurality of curvilinear struts comprising a degree of curvature and/or twist, and a spacing between adjacent curvilinear struts defining the cells of the transition section and the third stent cell pattern.

Example 5. The stent of example 4, further comprising the degree of curvature and/or twist adapted to be the same for each of the plurality of curvilinear struts.

Example 6. The stent of example 4, further comprising the spacing between adjacent curvilinear struts being constant or equal across the plurality of curvilinear struts.

Example 7. The stent of example 4, further comprising the degree of curvature and/or twist adapted to differ or vary for at least one of the plurality of curvilinear struts.

Example 8. The stent of example 5, wherein adjacent curvilinear struts are adapted to nest together when the stent is collapsed.

Example 9. The stent of example 6, wherein adjacent curvilinear struts are adapted to nest together when the stent is collapsed.

Example 10. The stent of example 7, wherein curvilinear struts are adapted to at least partially nest together when the stent is collapsed.

Example 11. The stent of example 4, further comprising the spacing between adjacent curvilinear struts varying for at least one pair of adjacent curvilinear struts.

Example 12. The stent of example 1, wherein the third stent cell pattern of the transition section further comprises a plurality of straight struts and a spacing between adjacent straight struts defining the cells of the transition section and the third stent cell pattern.

Example 13. The stent of example 12, wherein the plurality of straight struts are slanted with respect to the inner valve support section.

Example 14. The stent of example 13, wherein adjacent slanted straight struts are adapted to nest together when the stent is collapsed.

Example 15. The stent of example 1, further comprising the transition section comprising at least one expanded shape selected from the group consisting of: circular, oval, elliptical, or D-shaped.

Example 16. The stent of example 1, wherein the transition section comprises a flared profile comprising a diameter that is equal to or greater than an expanded maximum diameter of the outer section of the stent.

Example 17. The stent of example 1, wherein the transition section comprises a plurality of lobe-shaped elements.

Example 18. The stent of example 17, wherein the stent is adapted to treat a native heart valve comprising leaflets and wherein the number of lobe-shaped elements is matched to the number of native leaflets in the native valve.

Example 19. The collapsible and expandable stent of example 17, further comprising the lobe-shaped elements of the transition section adapted to direct blood flowing in the regurgitating upstream direction through the native valve across the native leaflets in a manner that reduces the closing volume and/or pressure required to close prosthetic leaflets attached within the inner valve support section.

Example 20. The stent of example 1, further comprising the outer section having a top section of an expanded shape selected from the group consisting of: flat, convex, concave or slanted.

Example 21. The stent of example 1, further comprising at least two slots defined and formed by adjacent struts forming the second cell structure pattern of the inner valve support section, each slot adapted for receiving an inner end of at least one of the prosthetic valve leaflets therethrough and further adapted to align, and maintain alignment, of the prosthetic valve leaflet within the inner valve support section.

Example 22. The stent of example 21, further comprising one or more eyelets through each adjacent strut defining and forming each of the at least two slots, each eyelet adapted for suturing the inner end of the prosthetic valve thereto.

Example 23. The stent of example 21, further comprising three eyelets through each adjacent strut that form and define each of the at least two slots.

Example 24. The stent of example 23, wherein the three eyelets in each adjacent strut define three pairs of eyelets, wherein a slot is positioned between the adjacent struts and the three pairs of eyelets defined by the adjacent struts.

Example 25. The stent of example 1, further comprising the outer section comprising a plurality of struts defining the first stent cell pattern; and at least one fabric attachment eyelet or aperture defined through at least one of the plurality of struts of the outer section.

Example 26. The stent according to example 1, wherein the stent comprises a prosthetic mitral valve or a prosthetic tricuspid valve.

Example 27. The stent according to example 1, wherein the outer section, the transition section and the inner valve support section comprise one continuous structure.

Example 28. A collapsible and expandable stent adapted to treat blood flow regurgitation in a native heart valve by allowing downstream blood flow and preventing upstream blood flow, the stent comprising an outer section; an inner valve support section extending radially upward into the outer section and comprising the first stent cell pattern or a second stent cell pattern, the inner valve support section supporting prosthetic valve leaflets attached therein; a transition section between the outer section and the inner valve support section, a boss section integrated with, or attached to, the outer section and extending downstream from the outer section and transition section.

Example 29. The stent of example 28, wherein the boss section comprising at least one expanded shape selected from the group consisting of: circular, D-shaped, oval, elliptical, and complementary and/or adaptable to the upper annular shape.

Example 30. The stent of example 28, wherein the boss section is contoured to complement the shape of the native heart valve.

Example 31. The stent of claim 28, further comprising the boss section extending downstream to a position that is selected from the group consisting of: above the annular plane, substantially co-planar with the annular plane, and slightly below the annular plane within the annulus.

Example 32. The stent of example 28, wherein the boss section is adapted to expand radially within an annular throat of the native heart valve.

Example 33. The stent of example 32, wherein the boss section is adapted to align the stent within the native heart valve and the prosthetic leaflets with native valve leaflets.

Example 34. The stent of example 32, wherein the boss section is adapted to mitigate perivalvular leakage within the native heart valve.

Example 35. A collapsible and expandable stent adapted to treat blood flow regurgitation in a native heart valve by allowing downstream blood flow and preventing upstream blood flow, the stent comprising an outer section; an inner valve support section extending radially upward into the outer section and comprising the first stent cell pattern or a second stent cell pattern, the inner valve support section supporting prosthetic valve leaflets attached therein; a transition section between the outer section and the inner valve support section, a boss section integrated with, or attached to, the transition section and extending downstream from the outer section and transition section.

Example 36. The stent of example 35, wherein the boss section comprising at least one expanded shape selected from the group consisting of: circular, D-shaped, oval, elliptical, and complementary and/or adaptable to the upper annular shape.

Example 37. The stent of example 35, wherein the boss section is contoured to complement the shape of the native heart valve.

Example 38. The stent of example 35, further comprising the boss section extending downstream to a position that is selected from the group consisting of: above the annular plane, substantially co-planar with the annular plane, and slightly below the annular plane within the annulus.

Example 39. The stent of example 35, wherein the boss section is adapted to expand radially within an annular throat of the native heart valve.

Example 40. The stent of example 39, wherein the boss section is adapted to align the stent within the native heart valve and the prosthetic leaflets with native valve leaflets.

Example 41. The stent of example 39, wherein the boss section is adapted to mitigate perivalvular leakage within the native heart valve.

## Claims

1. A collapsible and expandable stent (100) adapted to treat blood flow regurgitation in a mitral valve or a tricuspid valve of a heart by allowing downstream blood flow and preventing upstream blood flow, the stent comprising:
an outer section (102) comprising a first stent cell pattern;
an inner valve support section (104) extending radially upward into the outer section (102) and comprising a second stent cell pattern, the inner valve support section (104) supporting prosthetic valve leaflets attached therein;
a transition section (106) between the outer section (102) and the inner valve support section (104) comprising a third stent cell pattern that is different from the first stent cell pattern and the second stent cell pattern, wherein the transition section (106) turns radially inward to give rise to a radially upward extension of the inner valve support section (104) within the outer section (102);
wherein:
the transition section (106) further comprises a plurality of curvilinear struts (108') comprising a degree of curvature and/or twist;
a spacing between adjacent curvilinear struts (108') defines the third stent cell pattern of the transition section (106); and
adjacent curvilinear struts (108') are configured to nest together when the stent (100) is collapsed.

2. The stent (100) of claim 1 wherein the outer section (102) is round or oval.

3. The stent (100) of claim 1 or 2 wherein the inner valve support section (104) is cylindrical.

4. The stent (100) of claim 3 wherein a diameter of the inner valve support section (104) is not constant.

5. The stent (100) of claim 1 wherein, when the stent (100) is implanted in the mitral valve or the tricuspid valve, the inner valve support section (104) is adapted to support the prosthetic valve leaflets at a point located above a native annulus of the heart.

6. The stent (100) of claim 1 wherein, when the stent (100) is implanted in the mitral valve or the tricuspid valve, the inner valve support section (104) is adapted to support the prosthetic valve leaflets at a point disposed above native leaflets of the heart.

7. The stent (100) of claim 1 wherein the outer section (102), the inner valve support section (104) and the transition section (106) are constructed from one continuous structure.

8. The stent (100) of claim 1 wherein the transition section (106) comprises at least one expanded shape selected from the group consisting of: circular, oval, elliptical, or D-shaped.

9. The stent (100) of claim 1 wherein, when the stent (100) is implanted in the mitral valve or the tricuspid valve, the transition section (106) is located in the heart at a sub-annular position.

10. The stent (100) of claim 1 wherein a bottom section of the stent (100) is flat, convex, concave or slanted.

11. The stent (100) of claim 1 wherein a top of the outer section (102) is flat, convex, concave or slanted.

12. The stent (100) of any one of claims 1-11 further comprising a boss section (B).

13. The stent (100) of claim 12 wherein the boss section (B) extends away from the outer section (102).

14. The stent (100) of claim 12 or 13 wherein the boss section (B) is integrated with, or attached to, the transition section (106).

15. The stent (100) of any one of claims 12-14 wherein, when the stent is implanted in the mitral valve or the tricuspid valve, the boss section (B):
extends away from the outer section (102) in a downstream direction;
extends into an annular throat of an annulus of the heart; and
presses against an inner portion of the annular throat.
